# EUROPÄISCHE PATENTSCHRIFT

(11) **EP 0 339 416 B1**
(45) Veröffentlichungstag und Bekanntmachung des Hinweises auf die Patenterteilung: **26.05.1993**
(21) Anmeldenummer: 89106816.5
(22) Anmeldetag: 17.04.1989
(51) Int. Cl.: C07D 215/14, A61K 31/47

(54) **Gegebenenfalls alpha-substituierte 4-(Chinolin-2-yl-methoxy)phenylessigsäuren und -ester**
Optionally alpha-substituted 4-(quinolin-2-yl-methoxy)phenyl acetic acids and esters
Acides (quinolyl-2 méthoxy)-4 phénylacétiques et leurs esters éventuellement substitués en positions alpha

(30) Priorität: 29.04.1988 DE 3814504
(43) Veröffentlichungstag der Anmeldung: 02.11.1989
(73) Patentinhaber: BAYER AG, 51368 Leverkusen (DE)
(72) Erfinder: Mohrs, Klaus, Dr., D-5600 Wuppertal 1 (DE); Raddatz, Siegfried, Dr., D-5000 Köln 80 (DE); Fruchtmann, Romanis, D-5000 Köln 1 (DE); Kohlsdorfer, Christian, D-5042 Erftstadt (DE); Müller-Peddinghaus, Reiner, Prof.Dr., D-5060 Bergisch Gladbach 2 (DE)

(56) Entgegenhaltungen:
- EP-A- 0 110 405
- EP-A- 0 181 568
- US-A- 4 661 499

## Beschreibung

Die Erfindung betrifft neue gegebenenfalls α-substituierte 4-(Chinolin-2-yl-methoxy)phenylessigsäuren und deren Ester, Verfahren zu ihrer Herstellung und ihre Verwendung in Arzneimitteln.

In EP-A 181 568 werden 3-(Chinolin-2-yl-methoxy)phenyl-essigsäure und 2-[3-(Chinolin-2-yl-methoxy)phenyl]propionsäure, sowie deren Methyl- und ethylester mit antiinflammatorischer und antiallergischer Wirkung beschrieben.

US 4 661 499 betrifft α-hydroxyalkylsubstituierte Chinolylmethoxyphenyl-Derivate mit antiasthmatischer, antiinflammatorischer sowie antiallergischer Wirkung.

Es wurden neue gegebenenfalls α-substituierte 4-(Chinolin-2-yl-methoxy)phenylessigsäuren und deren Ester der allgemeinen Formel (I), 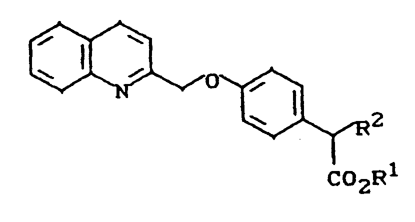
in welcher
- R¹: - für Wasserstoff, Alkyl mit 1 bis 6 Kohlenstoffatomen, Aralkylrest mit 1 bis 6 Kohlenstoffatomen im aliphatischen Teil und 6 bis 12 Kohlenstoffatomen im aromatischen Teil, Aryl mit 6 bis 12 Kohlenstoffatomen, oder
- für eine Gruppe der Formel

-CH₂-CO₂-R³

steht,

wobei
- R³: - für Wasserstoff, Alkyl mit 1 bis 6 Kohlenstoffatomen, Aralkylrest mit 1 bis 6 Kohlenstoffatomen im aliphatischen Teil und 6 bis 12 Kohlenstoffatomen im aromatischen Teil oder Aryl mit 6 bis 12 Kohlenstoffatomen steht
und
- R²: - für Wasserstoff, Alkyl mit 1 bis 6 Kohlenstofatomen, Alkenyl mit 2 bis 6 Kohlenstoffatomen oder Alkinyl mit 2 bis 6 Kohlenstoffatomen steht,
sowie deren Salze,
gefunden.

Im Vergleich zu den aus EP-A 181 568 bekannten metasubstituierten Verbindungen haben die erfindungsgemäßen Säuren und Ester der allgemeinen Formel (I) überraschenderweise eine höhere pharmakologische Wirkung, besonders nach peroraler Applikation.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze einwertiger Metalle wie die Alkalimethalle und die des Ammoniums. Bevorzugt werden Natrium-, Kalium- und Ammoniumsalze.

Es werden Verbindungen der allgemeinen Formel (I) bevorzugt,
worin
- R¹: - für Wasserstoff, C₁-C₆-Alkyl, Benzyl, Phenyl, oder
- für eine Gruppe der Formel

-CH₂-CO₂-R³

steht,

wobei
- R³: - für Wasserstoff, C₁-C₆-Alkyl, Phenyl oder Benzyl steht
und
- R²: - für Wasserstoff, C₁-C₆-Alkyl, verzweigtes C₁-C₆-Alkyl, C₂-C₆-Alkenyl, verzweigtes C₂-C₆-Alkenyl, C₂-C₆-Alkinyl oder verzweigtes C₂-C₆-Alkinyl steht,
sowie deren Salze.

Besonders bevorzugte Verbindungen der allgemeinen Formel (I) sind solche,
in denen
- R¹: - für Wasserstoff, Methyl, Ethyl, Benzyl, Phenyl, oder
- für eine Gruppe der Formel

-CH₂-CO₂-R³

steht,

wobei
- R³: - für Wasserstoff, Methyl, Ethyl, Phenyl oder Benzyl steht
und
- R²: - für Wasserstoff, Methyl, Ethyl, Propyl, Butyl, Pentyl, Hexyl, Isopropyl, Isobutyl, Isopentyl, 2-Butenyl, 2-Pentenyl, Allyl, 3,3-Dimethylallyl, 2-Butinyl, 2-Propinyl, 3-Pentinyl, 1-Methyl-2-butinyl oder 3-Hexinyl steht,
sowie deren Salze.

Beispielsweise seien folgende Wirkstoffe im einzelnen genannt:
4-(Chinolin-2-yl-methoxy)phenylessigsäuremethylester
2-[4-(Chinolin-2-yl-methoxy)phenyl]propionsäuremethylester
2-[4-(Chinolin-2-yl-methoxy]phenyl]buttersäuremethylester
2-[4-(Chinolin-2-yl-methoxy)phenyl]valeriansäuremethylester
2-[4-(Chinolin-2-yl-methoxy)phenyl]capronsäuremethylester
2-[4-(Chinolin-2-yl-methoxy)phenyl]heptansäuremethylester
2-[4-(Chinolin-2-yl-methoxy)phenyl]-5-methyl-4-hexensäuremethylester
E-2-[4-(Chinolin-2-yl-methoxy)phenyl]-4-hexensäuremethylester
2-[4-(Chinolin-2-yl-methoxy)phenyl]-4-hexinsäuremethylester
2-[4-(Chinolin-2-yl-methoxy)phenyl]isocapronsäuremethylester
2-[4-(Chinolin-2-yl-methoxy)phenyl]isovaleriansäuremethylester
2-[4-(Chinolin-2-yl-methoxy)phenyl]-5-octinsäuremethylester
4-(Chinolin-2-yl-methoxy)phenylessigsäure
2-[4-(Chinolin-2-yl-methoxy)phenyl]propionsäure
2-[4-(Chinolin-2-yl-methoxy)phenyl]buttersäure
2-[4-(Chinolin-2-yl-methoxy)phenyl]valeriansäure
2-[4-(Chinolin-2-yl-methoxyphenyl]capronsäure
2-[4-(Chinolin-2-yl-methoxy)phenyl]heptansäure
2-[4-(Chinolin-2-yl-methoxy)phenyl]-5-methyl-4-hexensäure
E-2-[4-(Chinolin-2-yl)methoxy)phenyl]-4-hexensäure
2-[4-(Chinolin-2-yl-methoxy)phenyl]-4-hexinsäure
2-[4-(Chinolin-2-yl-methoxy)phenyl]isocapronsäure
2-[4-(Chinolin-2-yl-methoxy)phenyl]isovaleriansäure
2-[4-(Chinolin-2-yl-methoxy)phenyl]capronsäuremethoxycarbonyl-methylester-Hydrochlorid
2-[4-(Chinolin-2-yl-methoxy)phenyl]capronsäurebenzyloxycarbonylmethylester
2-[4-(Chinolin-2-yl-methoxy)phenyl]capronsäurecarboxymethylester
4-(Chinolin-2-yl-methox)phenylessigsäuremethoxycarbonylmethylester
4-(Chinolin-2-yl-methoxy)phenylessigsäurebenzyloxycarbonylmethylester
4-(Chinolin-2-yl-methoxy)phenylessigsäurecarboxymethylester
2-[4-(Chinolin-2-yl-methoxy)phenyl]capronsäure-Natriumsalz
2-[4-(Chinolin-2-yl-methoxy)phenyl]capronsäureethylester
Außerdem wurde ein Verfahren zur Herstellung der gegebenenfalls α-substituierten 4-(Chinolin-2-yl-methoxy)phenylessigsäuren und -ester der allgemeinen Formel (I) 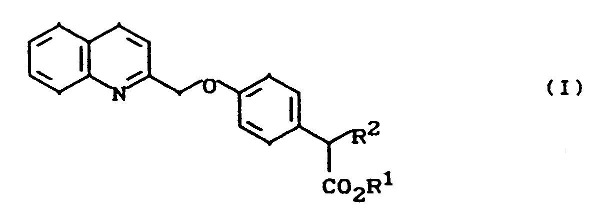
in welcher
R¹ und R² die oben angegebene Bedeutung haben
gefunden,
das dadurch gekennzeichnet ist, daß man

### [A] Phenole der allgemeinen Formel (II)

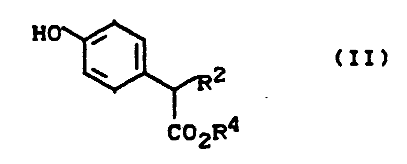

in welcher
R² die oben angegebene Bedeutung hat,
und
- R⁴: - für Alkyl, Arylalkyl, Aryl, oder
- für eine Gruppe der Formel

-CH₂-CO₂-R⁵

steht,

wobei
- R⁵: - für Alkyl, Aralkyl oder Aryl steht,
mit 2-Halogenmethylchinolin der Formel (III) 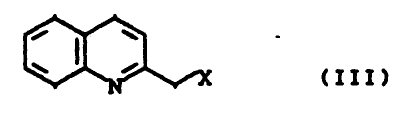
in der
- X: für Halogen steht,
umsetzt,
und im Fall der Säuren die Ester verseift,
oder indem man

### [B] Ester der allgemeinen Formel (IV)

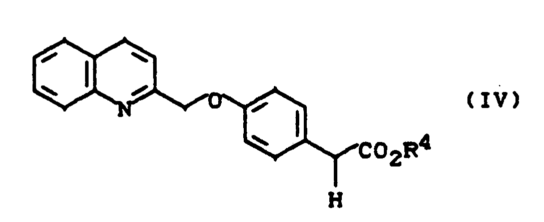

in der
R⁴ die oben angegebene Bedeutung hat,
mit Halogeniden der allgemeinen Formel (V)

R⁶ - X (V)

in welcher
- R⁶: - für Alkyl, Alkenyl oder Alkinyl steht
und
- X: - für Halogen steht,
alkyliert,
im Fall der Herstellung von Säuren die Ester verseift und gegebenenfalls durch Zugabe von Basen die Salze herstellt.

Die erfindungsgemäßen Verfahrensvarianten können durch die folgenden Formelschemata erläutert werden:

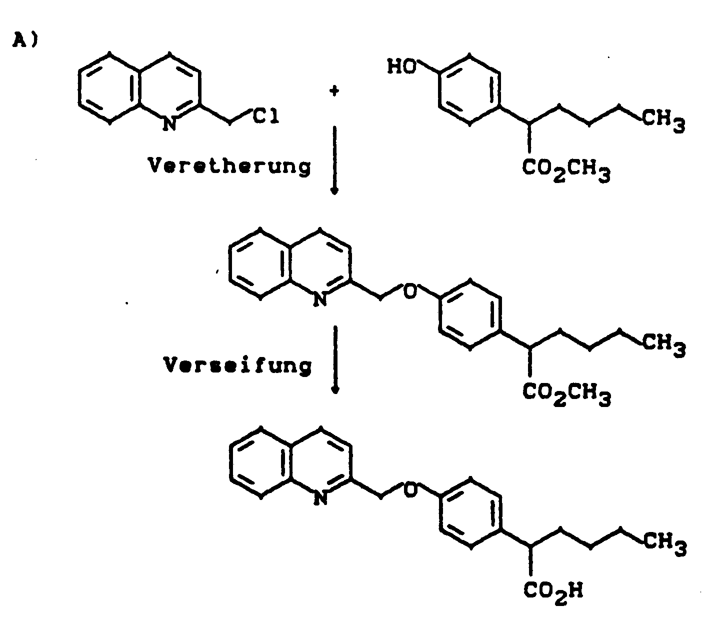

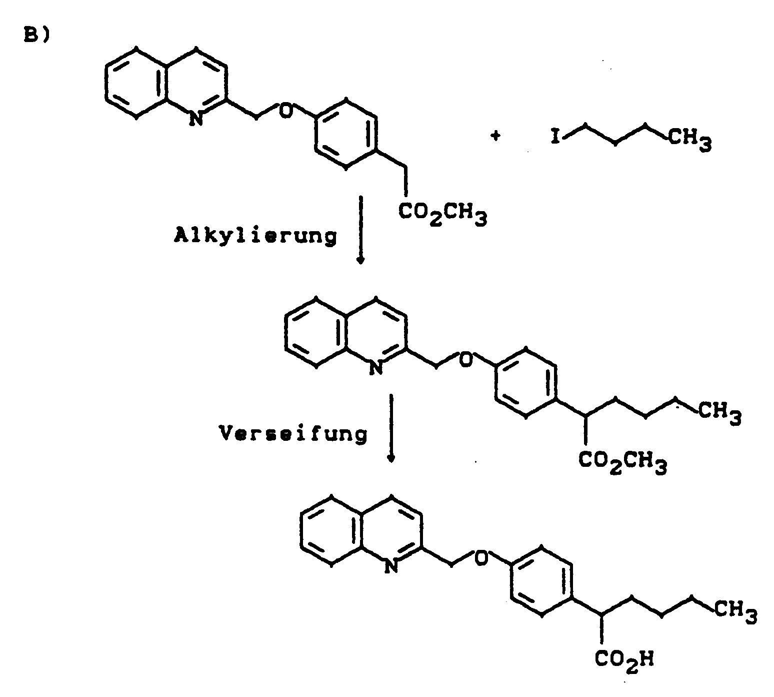

Die Veretherung kann in inerten organischen Lösemitteln gegebenenfalls in Anwesenheit einer Base durchgeführt werden.

Lösemittel für die erfindungsgemäßen Verfahren können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für das erfindunsgemäße Verfahren können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl(C₁-C₆)amine) wie Triethylamin, oder Heterocyclen wie Pyridin, Methylpiperidin, Piperidin oder Morpholin.

Es ist auch möglich als Basen Alkalimetalle, wie Natrium oder deren Hydride, wie Natriumhdyrid, einzusetzen.

Die Herstellung der erfindungsgemäßen Verbindungen der allgemeinen Formel (I) erfolgt im allgemeinen im Temperaturbereich von 0° C bis 150° C, bevorzugt von 10° C bis 100° C.

Die erfindungsgemäßen Verfahrensvarianten werden im allgemeinen bei Normaldruck durchgeführt. Es ist aber auch möglich, das Verfahren bei Unterdruck oder Überdruck durchzuführen (z.B. im Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgemeinen in einer Menge von 0,5 bis 5 mol, bevorzugt von 1 bis 3 mol bezogen auf das Halogenid eingesetzt.

Die Alkylierung der C-H aciden Verbindungen (Formel IV) erfolgt im allgemeinen in inerten Lösemitteln in Anwesenheit einer Base mit Alkylhalogeniden.

Als Lösemittel eignen sich hierbei, je nach Art des Alkylierungsmittels alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Dimethylformamid oder Hexamethylphosphorsäuretriamid, oder Gemische der genannten Lösemittel.

Als Basen eignen sich die üblichen basischen Verbindungen. Hierzu gehören bevorzugt Alkalihydride wie Natriumhydrid, Alkaliamide wie Natriumamid oder Lithiumdiisopropylamid, Alkalialkoholate wie Natriummethanolat, Natriumethanolat, Kaliummethanolat, Kaliumethanolat oder Kalium-tert.butylat, oder organische Amine wie Trialkylamine z.B. Triethylamin, oder lithiumorganische Verbindungen wie Butyllithium oder Phenyllithium.

Die Herstellung der erfindungsgemäßen Verbindungen erfolgt im allgemeinen im Temperaturbereich von 0° C bis 150° C, bevorzugt von 10° C bis 100° C.

Das erfindungsgemäße Verfahren wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgemeinen in einer Menge von 0,5 bis 5 mol, bevorzugt von 1 bis 3 mol bezogen auf das Halogenid eingesetzt.

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen im Temperaturbereich von 0° C bis +100° C, bevorzugt von +20° C bis +80° C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Die Ester der allgemeinen Formel (I), in welcher R¹ für eine Gruppe -CH₂-CO₂R³ steht und R³ die oben angegebene Bedeutung hat, können auch aus den entsprechenden Säuren der allgemeinen Formel (I) nach bekannten Methoden durch Veresterung mit α-Hydroxyessigsäureestern oder durch alkyliernde Veresterung mit α-Halogenessigsäureestern dargestellt werden.

Die Säuren wiederum können z.B. durch hydrolytische oder hydrogenolytische Spaltung der entsprechenden Benzylester nach bekannten Methoden dargestellt werden:

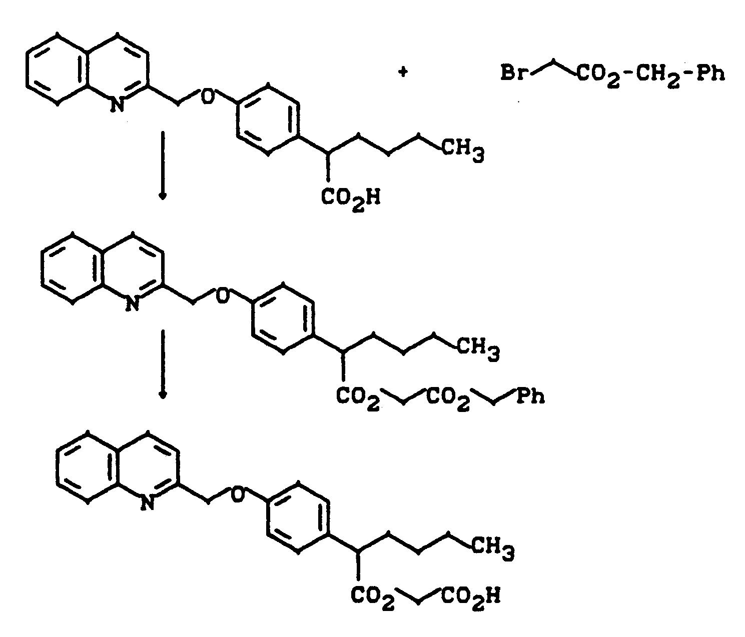

Die als Ausgangsverbindungen verwendeten Phenole der allgemeinen Formel (II) sind an sich bekannt und können aus den entsprechenden Ethern durch Abspaltung von Schutzgruppen nach üblichen Methoden dargestellt werden [Th. Greene: "Protective Groups in Organic Synthesis, J. Wiley & Sons, 1981, New York].

Die als Ausgangsverbindungen verwendeten Ester der allgemeinen Formel (IV) werden aus den bekannten 4-Hydroxyphenylessigsäureestern in einer Alkylierungsreaktion mit 2-Chlormethylchinolin hergestellt (analog Verfahrensvariante A).

Die Veresterung der Carbonsäuren erfolgt nach üblichen Methoden, indem man die Säuren in inerten Lösemitteln, gegebenenfalls in Anwesenheit einer Base, mit Alkylhalogeniden behandelt.

Als Basen eignen sich die üblichen organischen Amine. Hierzu gehören bevorzugt Alkylamine wie Triethylamin, Diisopropylamin, Dicyclohexylamin und Ethyldiisopropylamin.

Als Lösemittel eignen sich hierbei alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Dimethylformamid oder Gemische der genannten Lösemittel.

Die Herstellung der erfindungsgemäßen Verbindungen erfolgt im allgemeinen in einem Temperaturbereich von 0° C bis 150° C, bevorzugt von 10° C bis 100° C.

Das erfindungsgemäße Verfahren wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 mol Halogenid, bezogen auf 1 mol des Reaktionspartners ein. Die Base wird im allgmeinen in einer Menge von 0,5 bis 5 mol, bevorzugt von 1 bis 3 mol bezogen auf das Halogenid eingesetzt.

Im allgemeinen setzt man 0,01 bis 1, bevorzugt 0,05 bis 0,5 mol Katalysator, bezogen auf 1 mol Reaktionspartner ein.

Die hydrogenolytische Spaltung der Benzylester erfolgt nach üblichen Methoden, indem man die Benzylester in einem inerten Lösemittel in Anwesenheit eines Katalysators mit Wasserstoff-Gas hydriert.

Als Katalysatoren eignen sich die üblichen Metallkatalysatoren, die gegebenenfalls auf einem inerten Träger wie zum Beispiel Kohle in variablen Konzentrationen aufgebracht sind. Hierzu gehören bevorzugt Palladium, Nickel, Platin, besonders bevorzugt 5 bis 15% Palladium auf Aktivkohle.

Als Lösemittel eignen sich hierbei alle inerten organischen Lösemittel. Hierzu gehören bevorzugt Ether wie Diethylether, Dioxan oder Tetrahydrofuran, oder Kohlenwasserstoffe wie Benzol, Toluol oder Xylol, oder Alkohole wie Methanol, Ethanol oder Propanol, oder niedrigsiedende Ester wie Essigester, oder Amine wie Triethylamin, oder Gemische der genannten Lösemittel.

Die Herstellung der erfindungsgemäßen Verbindungen erfolgt im allgemeinen in einem Temperaturbereich von 0° C bis 150° C, bevorzugt von 10° C bis 100° C.

Das erfindungsgemäße Verfahren wird mit Wasserstoff im allgemeinen bei Normaldruck durchgeführt. Es ist aber auch möglich, das Verfahren bei Überdruck durchzuführen (z.B. in einem Bereich von 1 bis 10 bar).

Die erfindungsgemäßen Säuren und Ester können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysem, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, Metastasen und zur Cytoprotection im Gastrointestinaltrakt geeignet.

Die neuen Wirkstoffe können in an sich bekannter Weise unter Verwendung inerter nichttoxischer, pharmazeutischer geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Zubereitung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin-sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

Die erfindungsgemäßen Säuren und Ester können sowohl in der Humanmedizin als auch in der Veterinärmedizin angewendet werden.

### Herstellungsbeispiele

### Beispiel 1

### 2-(4-Methoxyphenyl)capronsäuremethylester

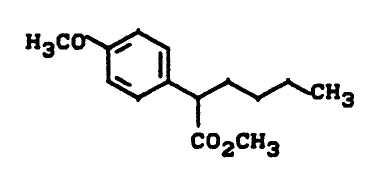

In 1 l Dimethylformamid werden unter Schutzgas 21,6 g Natriumhydrid suspendiert. 150 g 4-Methoxyphenylessigsäuremethylester, gelöst in 200 ml Dimethylformamid, werden unter Eiskühlung langsam zugetropft. Nach beendeter Wasserstoffentwicklung wird 1 h bei 25° C gerührt, anschließend 166 g Butyljodid unter Eiskühlung zugetropft und weitere 16 h bei 25° C gerührt. Das Lösemittel wird im Vakuum abgedampft, der Rückstand in Wasser aufgenommen und dreimal mit Essigester extrahiert. Nach Trocknen über Natriumsulfat wird das Lösemittel abgedampft und der Rückstand im Vakuum destilliert.
Ausbeute: 127g (65% der Theorie)
Siedepunkt: 101 - 105° C (0.1 mm)

### Beispiel 2

### 4-Benzyloxyphenylessigsäuremethylester

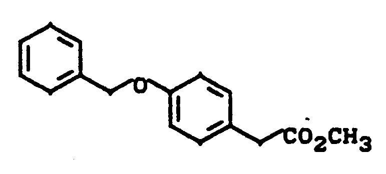

397 g 4-Hydroxyphenylessigsäuremethylester und 330 g Kaliumcarbonat werden in 2 l Dimethylformamid 1 h bei 50° C gerührt. Danach werden 302 g Benzylchlorid zugegeben und 15 h auf 50° C erwärmt. Nach Einengen im Vakuum wird der Rückstand zwischen Wasser und Essigester verteilt, die organische Phase über Natriumsulfat getrocknet und eingeengt. Das Produkt wird aus Methanol umkristallisiert.
Ausbeute: 511 g (83% der Theorie)
Festpunkt: 60° C

### Beispiel 3

### 2-(4-Benzyloxyphenyl)capronsäuremethylester

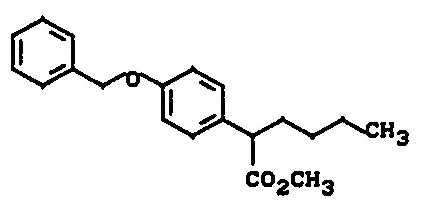

Die Darstellung erfolgt aus 102 g 4-Benzyloxyphenylessigsäuremethylester analog der Vorschrift aus Beispiel 1.
Ausbeute: 106 g (85% der Theorie)
Siedepunkt: 180° C (0.1 mm) (Kugelrohr)

### Beispiel 4

### 2-(4-Hydroxyphenyl)capronsäuremethylester

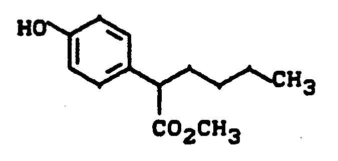

a) 83 g 2-(4-Methoxyphenyl)capronsäuremethylester werden in 750 ml Dichlormethan gelöst. Unter Schutzgas werden 360 ml einer 1 molaren Bortribromid-Dichlormethan-Lösung bei -75° C zugetropft. Nach langsamen Erwärmen wird 16 h bei 25° C gerührt. Nach Abdampfen des Lösemittels wird der Rückstand vorsichtig mit 1 l Methanol und 250 ml Wasser versetzt und 4 h unter Rückfluß erwärmt. Nach Einengen wird dreimal mit Dichlormethan extrahiert, eingedampft und der Rückstand im Vakuum destilliert.
   Ausbeute: 58,5 g (75% der Theorie)
   Siedepunkt: 130 - 135° C (0.1 mm)
b) 75 g 2-(4-Benzyloxyphenyl)capronsäuremethylester werden unter Zusatz von 1 g Palladium-Katalysator (5%ig auf Kohle) in 900 ml Methanol und 100 ml Triethylamin bei Normaldruck hydriert. Nach Aufnahme der theoretischen Menge Wasserstoff wird vom Katalysator abfiltriert, eingeengt und der Rückstand destilliert.
   Ausbeute: 44 g (82% der Theorie)

### Beispiel 5

### 4-(Chinolin-2-yl-methoxy)phenylessigsäuremethylester

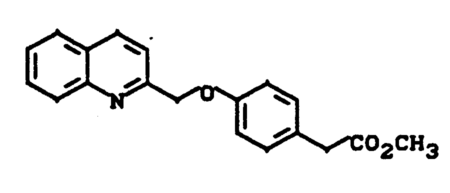

Die Darstellung erfolgt aus 200 g 4-Hydroxyphenylessigsäuremethylester und 214 g 2-Chlormethylchinolin analog der Vorschrift von Beispiel 2.
Ausbeute: 293 g (79% der Theorie)
Festpunkt: 71-73° C

### Beispiel 6

### 2-[4-(Chinolin-2-yl-methoxy)phenyl]propionsäuremethylester

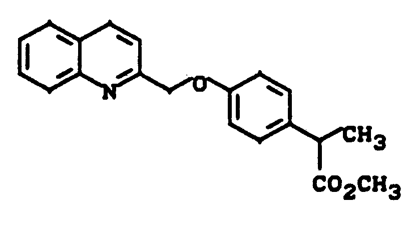

Die Darstellung erfolgt aus 15,4 g 4-(Chinolin-2-yl-methoxy)phenylessigsäuremethylester und 7,8 g Methyljodid analog der Vorschrift von Beispiel 1.
Ausbeute: 7,8 g (49% der Theorie)
Festpunkt: 187 - 190° C (0,5 x 1,5-Naphthalindisulfonsäure-Salz).

### Beispiel 7

### 2-[4-(Chinolin-2-yl-methoxy)phenyl]buttersäuremethylester

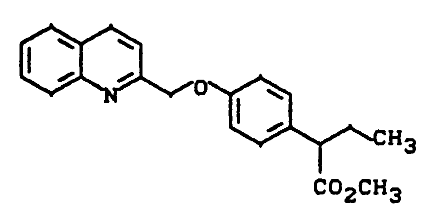

Die Darstellung erfolgt aus 15,4 g 4-(Chinolin-2-yl-methoxy)phenylessigsäuremethylester und 8,6 g Ethyljodid analog der Vorschrift von Beispiel 1.
Ausbeute: 7,8 g (47% der Theorie)
Festpunkt: 53 - 56° C

### Beispiel 8

### 2-[4-(Chinolin-2-yl-methoxy)phenyl]valeriansäuremethylester

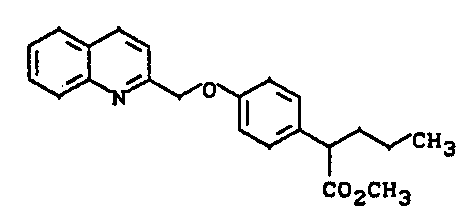

Die Darstellung erfolgt aus 15,4 g 4-Chinolin-2-yl-methoxy)phenylessigsäuremethylester und 5,8 g Propylbromid analog der Vorschrift von Beispiel 1.
Ausbeute: 7,9 g (45% der Theorie)
Festpunkt: 50 - 52° C

### Beispiel 9

### 2-[4-(Chinolin-2-yl-methoxy)phenyl]capronsäuremethylester

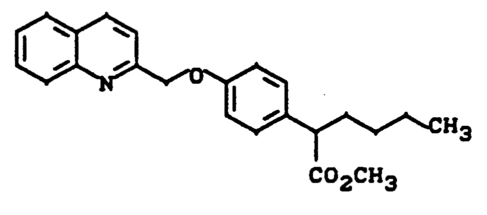

Die Darstellung erfolgt aus 56 g 2-(4-Hydroxyphenyl)capronsäuremethylester und 44 g 2-Chlormethylchinolin analog der Vorschrift von Beispiel 2.
Ausbeute: 77 g (85% der Theorie)
Festpunkt: 144 - 146° C (Hydrochlorid)

### Beispiel 10

### 2-[4-(Chinolin-2-yl-methoxy)-phenyl]heptansäuremethylester

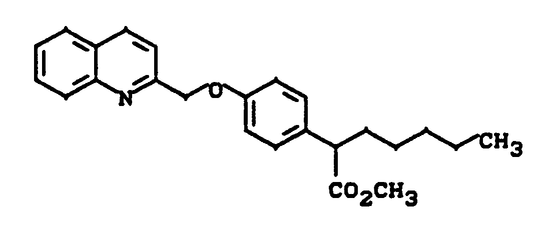

Die Darstellung erfolgt aus 15,4 g 4-(Chinolin-2-yl-methoxy)phenylessigsäuremethylester und 10,9 g Pentyljodid analog der Vorschrift von Beispiel 1.
Ausbeute: 9,8 g (52% der Theorie)
Festpunkt: 41° C

### Beispiel 11

### 2-[4-[Chinolin-2-yl-methoxy)phenyl]-5-methyl-4-hexensäuremethylester

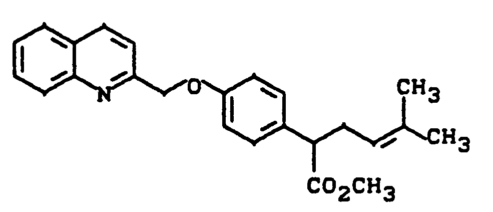

Die Darstellung erfolgt aus 15,4 g 4-(Chinolin-2-yl-methoxy)phenylessigsäuremethylester und 8,2 g 1-Brom-3-methyl-2-buten analog dar Vorschrift von Beispiel 1.
Ausbeute: 16 g (86% der Theorie)
Festpunkt: 54 - 56°C

### Beispiel 12

### E-2-[4-(Chinolin-2-yl-methoxy)phenyl]-4-hexensäuremethylester

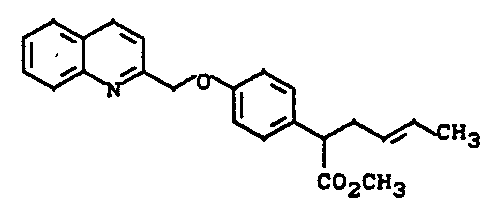

Die Darstellung erfolgt aus 15,4 g 4-(Chinolin-2-yl-methoxy)phenylessigsäuremethylester und 7,4 g trans-1-Brom-2-buten analog der Vorschrift von Beispiel 1.
Ausbeute: 12,2 g (68% der Theorie)
Festpunkt: 47°C

### Beispiel 13

### 2-[4-(Chinolin-2-yl-methoxy)phenyl]-4-hexinsäuremethylester

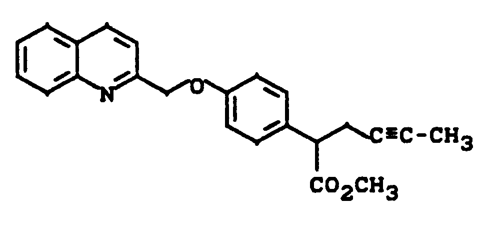

Die Darstellung erfolgt aus 15,4 g 4-(Chinolin-2-yl-methoxy)phenylessigsäuremethylester und 7,32 g 1-Brom-2-butin analog der Vorschrift von Beispiel 1.
Ausbeute: 10,2 g (57% der Theorie)
Festpunkt: 53° C

### Beispiel 14

### 2-[4-(Chinolin-2-yl-methoxy)phenyl]isocapronsäuremethylester

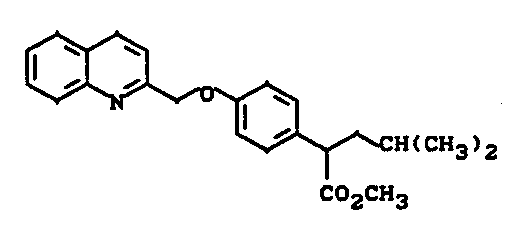

Die Darstellung erfolgt aus 15,4 g 4-(Chinolin-2-yl-methoxy)phenylessigsäuremethylester und 5,1 g Isobutylchlorid analog der Vorschrift von Beispiel 1.
Ausbeute: 8,0 g (44% der Theorie)
Festpunkt: 62° C.

### Beispiel 15

### 2-[4-(Chinolin-2-yl-methoxy)phenyl]isovaleriansäuremethylester

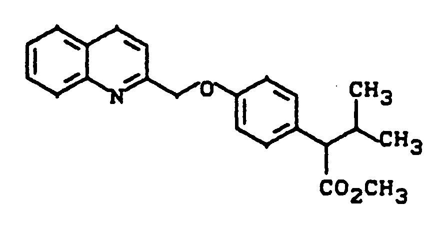

Die Darstellung erfolgt aus 15,4 g 4-(Chinolin-2-yl-methoxy)phenylessigsäuremethylester und 9,4 g Isopropyliodid analog der Vorschrift von Beispiel 1.
Ausbeute: 13 g (74% der Theorie)
Festpunkt: 69 - 71° C.

### Beispiel 16

### 4-(Chinolin-2-yl-methoxy)phenylessigsäure

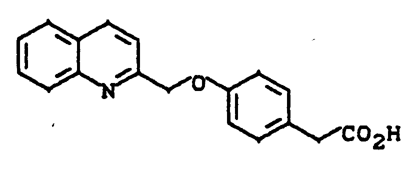

49 g 4-(Chinolin-2-yl-methoxy)phenylessigsäuremethylester werden in 250 ml Methanol und 192 ml 1 normaler Natronlauge 5 h bei 25° C gerührt. Unter Eiskühlung wird mit konzentrierter Salzsäure angesäuert. Das ausgefallene Produkt wird abgesauft, getrocknet und aus Aceton umkristallisiert.
Ausbeute: 44,6 g (95% der Theorie)
Festpunkt: 158 - 159° C

### Beispiel 17

### 2-[4-(Chinolin-2-yl-methoxy)phenyl]propionsäure

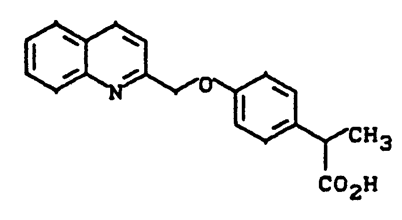

Die Darstellung erfolgt aus 5,3 g 2-[4-(Chinolin-2-yl-methoxy)phenyl]propionsäuremethylester analog der Vorschrift von Beispiel 16.
Ausbeute: 4 g (78% der Theorie)
Festpunkt: 145° C

### Beispiel 18

### 2-[4-(Chinolin-2-yl-methoxy)phenyl]buttersäure

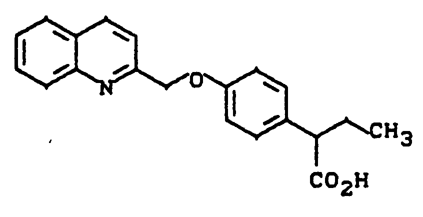

Die Darstellung erfolgt aus 5,1 g 2-[4-(Chinolin-2-yl-methoxy)phenyl]buttersäuremethylester analog der Vorschrift von Beispiel 16.
Ausbeute: 3,6 g (73% der Theorie)
Festpunkt: 153° C.

### Beispiel 19

### 2-[4-(Chinolin-2-yl-methoxy)phenyl]valeriansäure

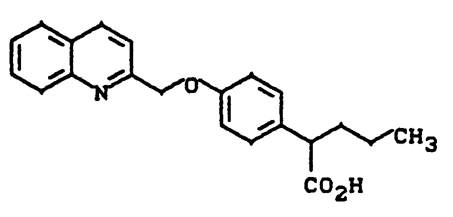

Die Darstellung erfolgt aus 5,3 g 2-[4-(Chinolin-2-yl-methoxy)phenyl]valeriansäuremethylester analog der Vorschrift von Beispiel 16.
Ausbeute: 3,1 g (61% der Theorie)
Festpunkt: 135° C

### Beispiel 20

### 2-[4-(Chinolin-2-yl-methoxy)phenyl]capronsäure

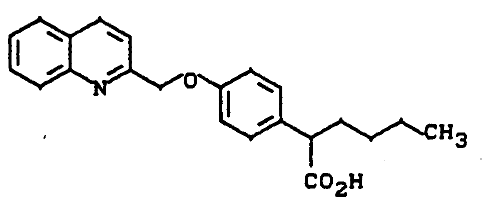

Die Darstellung erfolgt aus 91 g 2-[4-(Chinolin-2-yl-methoxy)phenyl]capronsäuremethylester analog der Vorschrift von Beispiel 16.
Ausbeute: 64,5 g (74% der Theorie)
Festpunkt: 131 - 132° C

### Beispiel 21

### 2-[4-(Chinolin-2-yl-methoxy)phenyl]heptansäure

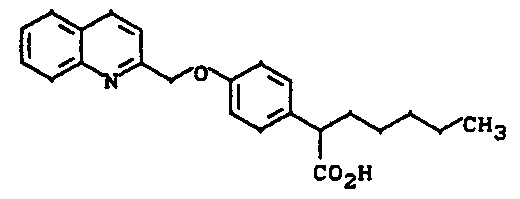

Die Darstellung erfolgt aus 7,2 g 2-[4-(Chinolin-2-yl-methoxy)phenyl]heptansäuremethylester analog der Vorschrift von Beispiel 16.
Ausbeute: 5,5 g (76% der Theorie)
Festpunkt: 75° C

### Beispiel 22

### 2-[4-(Chinolin-2-yl-methoxy)phenyl]-5-methyl-4-hexensäure

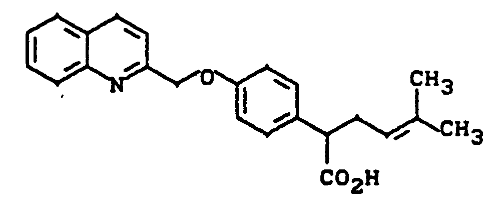

Die Darstellung erfolgt aus 13,7 g 2-[4-(Chinolin-2-yl-methoxy)phenyl]-5-methyl-4-hexensäuremethylester analog der Vorschrift von Beispiel 16.
Ausbeute: 8,4 g (64% der Theorie)
Festpunkt: 114° C

### Beispiel 23

### E-2-[4-(Chinolin-2-yl-methoxy)phenyl]-4-hexensäure

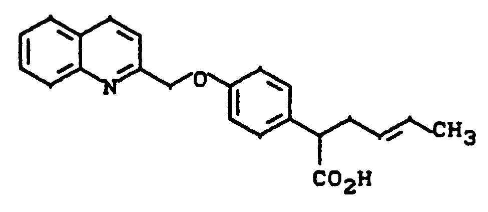

Die Darstellung erfolgt aus 9,7 g E-2-[4-(Chinolin-2-yl-methoxy)phenyl]-4-hexensäuremethylester analog der Vorschrift von Beispiel 16.
Ausbeute: 9,1 g (98% der Theorie)
Festpunkt: 137° C.

### Beispiel 24

### 2-[4-(Chinolin-2-yl-methoxy)phenyl]-4-hexinsäure

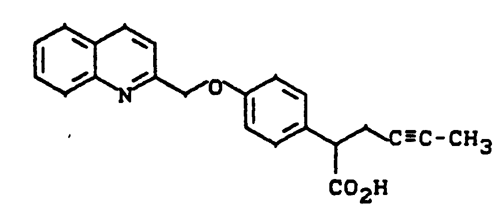

Die Darstellung erfolgt aus 6,7 g 2-[4-(Chinolin-2-yl-methoxy)phenyl]-4-hexinsäuremethylester analog der Vorschrift von Beispiel 16.
Ausbeute: 4,0 g (62% der Theorie)
Festpunkt: 177° C

### Beispiel 25

### 2-[4-(Chinolin-2-yl-methoxy)phenyl]isocapronsäure

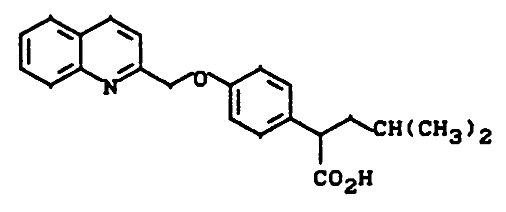

Die Darstellung erfolgt aus 5,8 g 2-4-(Chinolin-2-yl-methoxy)phenyl]isocapronsäuremethylester analog der Vorschrift von Beispiel 16.
Ausbeute: 4,2 g (75% der Theorie)
Festpunkt: 117° C

### Beispiel 26

### 2-[4-(Chinolin-2-yl-methoxy)phenyl]isovaleriansäure

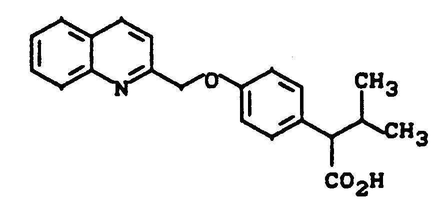

Die Darstellung erfolgt aus 11,7 g 2-[4-(Chinolin-2-yl-methoxy)phenyl]isovaleriansäuremethylester analog der Vorschrift von Beispiel 16.
Ausbeute: 10,6 g (95% der Theorie)
Festpunkt: 173° C

### Beispiel 27

### 2-[4-(Chinolin-2-yl-methoxy)phenyl]capronsäuremethoxycarbonylmethylester-Hydrochlorid

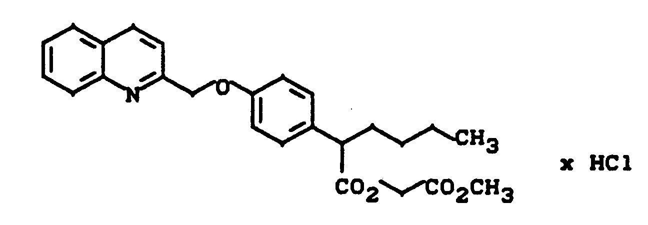

1,7 g 2-[4-(Chinolin-2-yl-methyloxy)phenyl]capronsäure, 0,84 g Bromessigsäuremethylester und 1 g Dicyclohexylamin werden in 30 ml Tetrahydrofuran 16 h zum Sieden erhitzt. Nach Abkühlen auf 0° C wird vom ausgefallenen Salz abfiltriert und das Lösemittel im Vakuum abgedampft. Der Rückstand wird in Ether aufgenommen und mit etherischer Chlorwasserstoff-Lösung versetzt. Das Produkt fällt aus, wird abfiltriert und getrocknet.
Ausbeute: 2 g (90% der Theorie)
Festpunkt: 70 - 73° C

### Beispiel 28

### 2-[4-(Chinolin-2-yl-methoxy)phenyl]capronsäurebenzyloxycarbonylmethylester

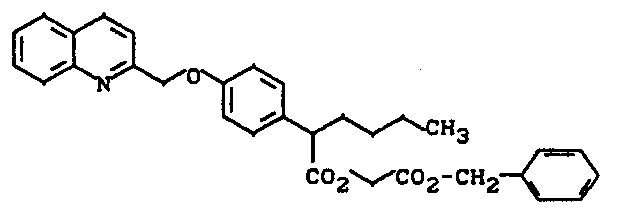

Die Darstellung erfolgt aus 7 g 2-[4-(Chinolin-2-yl-methoxy)phenyl]capronsäure und 5 g Bromessigsäurebenzylester analog der Vorschrift von Beispiel 27.
Ausbeute: 9,5 g (95% der Theorie)
Festpunkt: 68-69° C

### Beispiel 29

### 2-[4-(Chinolin-2-yl-methoxy)phenyl]capronsäurecarboxymethylester

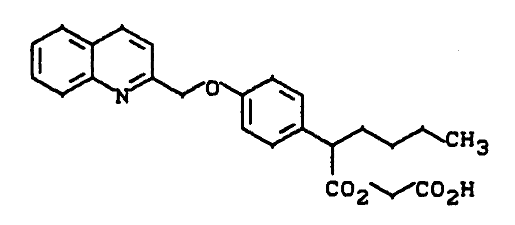

8,5 g 2-[4-(Chinolin-2-yl-methoxy)phenyl]capronsäurebenzyloxycarbonylmethylester werden in 150 ml Essigester und 15 ml Triethylamin gelöst, 0,9 g Palladium-Katalysator (10%ig auf Kohle) zugegeben und bei Normaldruck bei 25° C hydriert. Nach Aufnahme der theoretischen Menge Wasserstoff wird der Katalysator abfiltriert. Nach Einengen im Vakuum wird zwischen Essigester/Wasser verteilt, die organische Phase getrocknet und eingeengt.
Ausbeute: 4,3 g (62% der Theorie)
Festpunkt: 142 - 144° C (Hydrochlorid)

### Beispiel 30

### 4-(Chinolin-2-yl-methoxy)phenylessigsäuremethoxy-carbonylmethylester

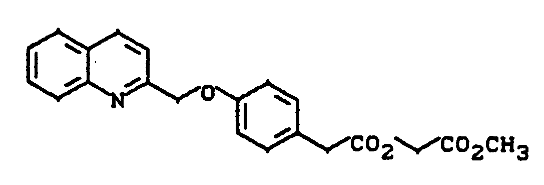

Die Darstellung erfolgt aus 7,3 g 4-(Chinolin-2-yl-methoxy)phenylessigsäure und 4,2 g Bromessigsäuremethylester analog der Vorschrift von Beispiel 27.
Ausbeute: 7,1 g (78% der Theorie)
Festpunkt: 82 - 83,5° C

### Beispiel 31

### 4-(Chinolin-2-yl-methoxy)phenylessigsäurebenzyloxycarbonylmethylester

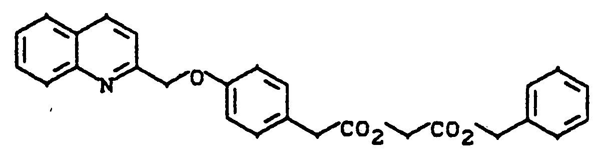

Die Darstellung erfolgt aus 7,3 g 4-(Chinolin-2-yl-methoxy)phenylessigsäure und 6,3 g Bromessigsäurebenzylester analog der Vorschrift von Beispiel 27.
Ausbeute: 6,3 g (57% der Theorie)
Festpunkt: 60 - 62° C

### Beispiel 32

### 4-(Chinolin-2-yl-methoxy)phenylessigsäurecarboxymethylester

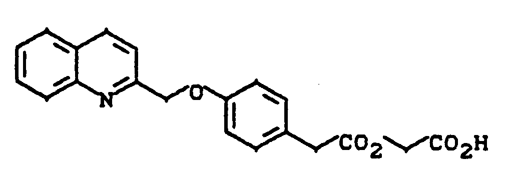

Die Darstellung erfolgt aus 5,7 g 4-(Chinolin-2-yl-methoxy)phenylessigsäurebenzyloxycarbonylmethylester analog der Vorschrift von Beispiel 29.
Ausbeute: 3,7 g (81% der Theorie)
Festpunkt: 148 - 149° C

### Beispiel 33

### 2-[4-(Chinolin-2-yl-methoxy)phenyl]capronsäure-Natriumsalz

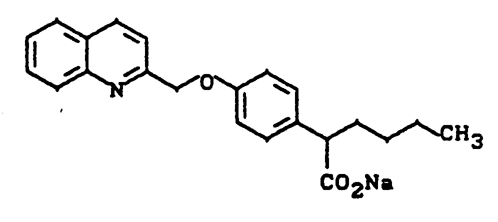

3,5 g 2-[4-(Chinolin-2-yl-methyloxy)phenyl]capronsäure werden in 40 ml Methanol gelöst. Nach Zugabe von 10 ml 1 normaler Natronlauge (equimolare Menge) wird bis zum Trockenen eingeengt und bei 100° C im Vakuum getrocknet.
Ausbeute: quantitativ
Festpunkt: 187 - 193° C

### Beispiel 34

### 2-[4-(Chinolin-2-yl-methoxy)phenyl]capronsäureethylester

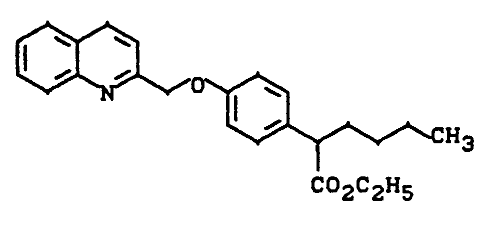

Die Darstellung erfolgt analog der Vorschrift von Beispiel 2 aus 36 g Chlormethylchinolin und 40 g 2-(4-Hydroxyphenyl)capronsäureethylester
Ausbeute: 52 g (81% der Theorie)
Festpunkt: 48° C

### Anwendungsbeispiel

### Beispiel 35

Im Vergleich mit der bekannten Substanz 2-[3-(1-Hydroxyhexyl)phenoxymethyl]chinolin [vgl. EP-A 110 405]) zeigen die erfindungsgemäßen Verbindungen eine deutlich stärkere pharmakologische Wirksamkeit.

Die pharmakologischen Wirkdaten der erfindungsgemäßen Substanzen werden durch folgende Methode bestimmt:

Als Maß für die Lipoxygenase-Hemmung wurde die Freisetzung von Leukotrien B₄ (LTB₄) an polymorphkernigen Rattenleukozyten (PMN) nach Zugabe von Substanzen und Ca-Ionophor mittels reverse phase HPLC nach Borgeat, P. et al., Proc. Nat. Acad. Sci. 76, 2148 - 2152 (1979) bestimmt. Die in vivo-Aktivität der Verbindungen wurde mit dem Mäuseohr-Entzündungsmodell nach Young, J.M. et al., J. of Investigative Dermatology 82, 367 - 371 (1984) nachgewiesen.

In den Tabellen 1 und 2 sind beispielhaft die nach diesen Tests erzielten Werte einiger erfindungsgemäßer Verbindungen aufgeführt:

**Tabelle 1**

| Beispiel | LO-Hemmung IC₅₀ (µM) |
|---|---|
| Nr. 20 | 0,055 |
| Nr. 27 | 0,063 |
| Nr. 29 | 0,055 |
| 2-[[3-(1-Hydroxyhexyl)phenoxy]methyl]chinolin | 0,2 |

**Tabelle 2**

| Beispiel | Entzündungshemmung % 100 mg/kg p.o. |
|---|---|
| Nr. 10 | 61 |
| Nr. 12 | 59 |
| Nr. 20 | 68 |
| Nr. 22 | 77 |
| Nr. 29 | 72 |
| 2-[[3-(1-Hydroxyhexyl)phenoxy]methyl]chinolin | 24 |

## Patentansprüche (Patentansprüche für folgende(n) Vertragsstaat(en): AT, BE, CH, DE, FR, GB, GR, IT, LI, NL, SE)

1. Gegebenenfalls α-substituierte [4-(Chinolin-2-yl-methoxy)phenyl]-essigsäuren und -ester der allgemeinen Formel 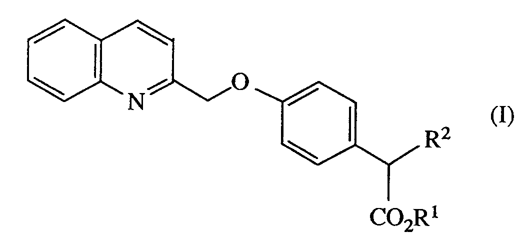 in welcher
R¹ - für Wasserstoff, Alkyl mit 1 bis 6 Kohlenstoffatomen, Aralkylrest mit 1 bis 6 Kohlenstoffatomen im aliphatischen Teil und 6 bis 12 Kohlenstoffatomen im aromatischen Teil, Aryl mit 6 bis 12 Kohlenstoffatomen, oder
- für eine Gruppe der Formel
-CH₂-CO₂-R³
steht,
wobei
R³ - für Wasserstoff, Alkyl mit 1 bis 6 Kohlenstoffatomen, Aralkylrest mit 1 bis 6 Kohlenstoffatomen im aliphatischen Teil und 6 bis 12 Kohlenstoffatomen im aromatischen Teil oder Aryl mit 6 bis 12 Kohlenstoffatomen steht
und
R² - für Wasserstoff, Alkyl mit 1 bis 6 Kohlenstofatomen, Alkenyl mit 2 bis 6 Kohlenstoffatomen oder Alkinyl mit 2 bis 6 Kohlenstoffatomen steht,
sowie deren Salze.

2. Gegebenenfalls α-substituierte [4-(Chinolin-2-yl-methoxy)phenyl]-essigsäuren und -ester nach Anspruch 1,
worin
R¹ - für Wasserstoff, C₁-C₆-Alkyl, Benzyl, Phenyl, oder
- für eine Gruppe der Formel
-CH₂-CO₂-R³
steht,
wobei
R³ - für Wasserstoff, C₁-C₆-Alkyl, Phenyl oder Benzyl steht
und
R² - für Wasserstoff, C₁-C₆-Alkyl, verzweigtes C₁-C-₆Alkyl, C₂-C₆-Alkenyl, verzweigtes C₂-C₆-Alkenyl, C₂-C₆-Alkinyl oder verzweigtes C₂-C₆-Alkinyl steht,
sowie deren Salze.

3. Gegebenenfalls α-substituierte [4-(Chinolin-2-yl-methoxy)phenyl]-essigsäuren und -ester nach den Ansprüchen 1 und 2,
worin
R¹ - für Wasserstoff, Methyl, Ethyl, Benzyl, Phenyl, oder
- für eine Gruppe der Formel
-CH₂-CO₂-R³
steht,
wobei
R³ - für Wasserstoff, Methyl, Ethyl, Phenyl oder Benzyl steht
und
R² - für Wasserstoff, Methyl, Ethyl, Propyl, Butyl, Pentyl, Hexyl, Isopropyl, Isobutyl, Isopentyl, 2-Butenyl, 2-Pentenyl, Allyl, 3,3-Dimethylallyl, 2-Butinyl, 2-Propinyl, 3-Pentinyl, 1-Methyl-2-butinyl oder 3-Hexinyl steht,
sowie deren Salze.

4. 4-(Chinolin-2-yl-methoxy)phenyl-essigsäure der Formel 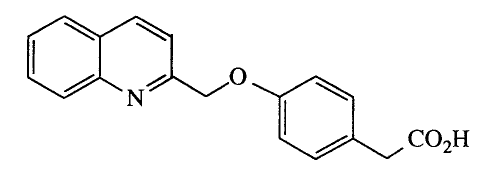 und deren Salze nach den Ansprüchen 1 bis 3.

5. Gegebenenfalls α-substituierte [4-(Chinolin-2-yl-methoxy)phenyl]-essigsäuren und -ester nach den Ansprüchen 1 bis 3 zur therapeutischen Behandlung.

6. Verfahren zur Herstellung von gegebenenfalls α-substituierten [4-(Chinolin-2-yl-methoxy)phenyl]-essigsäuren und -estern der Formel 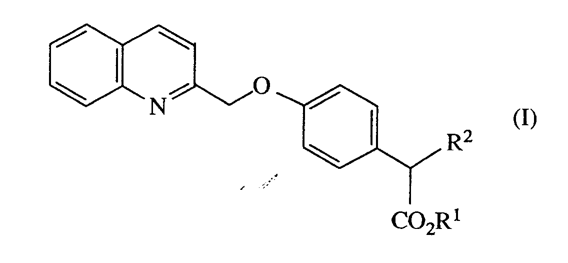 in welcher R¹ und R² die in Anspruch 1 angegebene Bedeutung haben, dadurch gekennzeichnet, daß man
[A] Phenole der allgemeinen Formel (II) 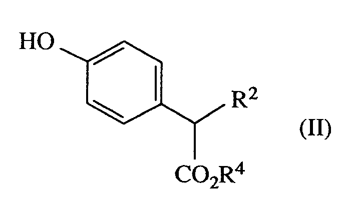 in welcher
R² die oben angegebene Bedeutung hat,
und
R⁴ - für Alkyl, Arylalkyl, Aryl, oder
- für eine Gruppe der Formel
-CH₂-CO₂-R⁵
steht,
wobei
R⁵ - für Alkyl, Aralkyl oder Aryl steht,
mit 2-Halogenmethylchinolin der Formel (III) 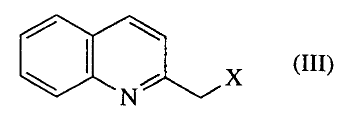 in der
X für Halogen steht,
umsetzt,
und im Fall der Säuren die Ester verseift,
oder indem man
[B] Ester der allgemeinen Formel (IV) 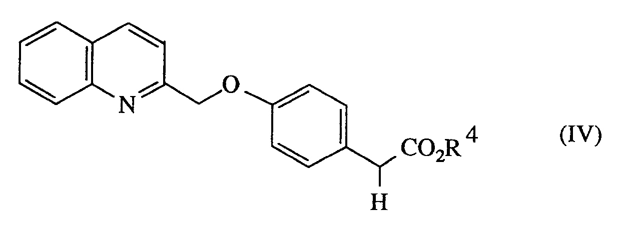 in der
R⁴ die oben angegebene Bedeutung hat,
mit Halogeniden der allgemeinen Formel (V)
R⁶ - X (V)
in welcher
R⁶ - für Alkyl, Alkenyl oder Alkinyl steht
und
X - für Halogen steht,
alkyliert,
im Fall der Herstellung von Säuren die Ester verseift und gegebenenfalls durch Zugabe von Basen die Salze herstellt.

7. Arzneimittel, enthaltend gegebenenfalls α-substituierte [4-(Chinolin-2-yl-methoxy)phenyl]-essigsäuren und -ester nach den Ansprüchen 1 bis 3.

8. Arzneimittel nach Anspruch 7, enthaltend 0,5 bis 90 Gew.-% gegebenenfalls α-substituierten [4-(Chinolin-2-yl-methoxy)phenyl]-essigsäuren und -ester bezogen auf die Zubereitung.

9. Verwendung von gegebenenfalls α-substituierten [4-(Chinolin-2-yl-methoxy)phenyl]-essigsäuren und -estern nach den Ansprüchen 1 bis 3 zur Herstellung von Arzneimitteln.

10. Verwendung nach Anspruch 9 zur Herstellung von Lipoxygenase-Hemmern.

## Patentansprüche (Patentansprüche für folgende(n) Vertragsstaat(en): ES)

1. Verfahren zur Herstellung von gegebenenfalls α-substituierten [4-(Chinolin-2-yl-methoxy)phenyl]-essigsäuren und -estern der allgemeinen Formel 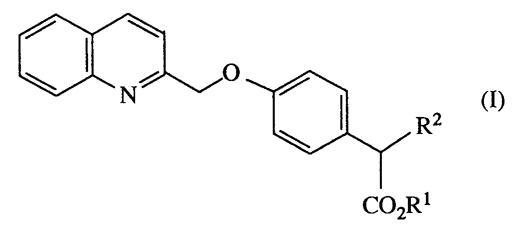 in welcher
R¹ - für Wasserstoff, Alkyl mit 1 bis 6 Kohlenstoffatomen, Aralkylrest mit 1 bis 6 Kohlenstoffatomen im aliphatischen Teil und 6 bis 12 Kohlenstoffatomen im aromatischen Teil, Aryl mit 6 bis 12 Kohlenstoffatomen, oder
- für eine Gruppe der Formel
-CH₂-CO₂-R³
steht,
wobei
R³ - für Wasserstoff, Alkyl mit 1 bis 6 Kohlenstoffatomen, Aralkylrest mit 1 bis 6 Kohlenstoffatomen im aliphatischen Teil und 6 bis 12 Kohlenstoffatomen im aromatischen Teil oder Aryl mit 6 bis 12 Kohlenstoffatomen steht
und
R² - für Wasserstoff, Alkyl mit 1 bis 6 Kohlenstofatomen, Alkenyl mit 2 bis 6 Kohlenstoffatomen oder Alkinyl mit 2 bis 6 Kohlenstoffatomen steht,
sowie deren Salze, dadurch gekennzeichnet, daß man
[A] Phenole der allgemeinen Formel (II) 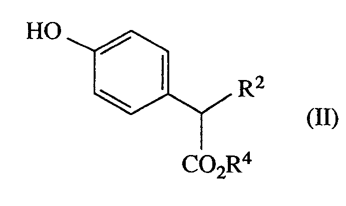 in welcher
R² die oben angegebene Bedeutung hat,
und
R⁴ - für Alkyl, Arylalkyl, Aryl, oder
- für eine Gruppe der Formel
-CH₂-CO₂-R⁵
steht,
wobei
R⁵ - für Alkyl, Aralkyl oder Aryl steht,
mit 2-Halogenmethylchinolin der Formel (III) 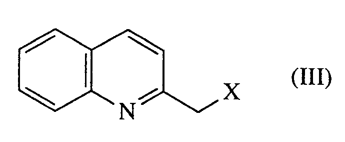 in der
X für Halogen steht,
umsetzt,
und im Fall der Sauren die Ester verseift,
oder indem man
[B] Ester der allgemeinen Formel (IV) 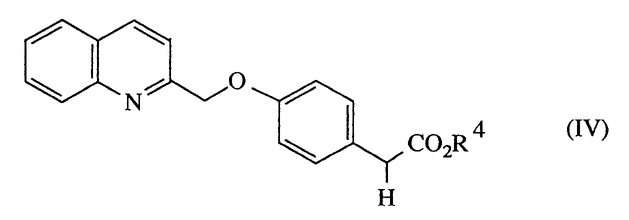 in der
R⁴ die oben angegebene Bedeutung hat,
mit Halogeniden der allgemeinen Formel (V)
R⁶-X (V)
in welcher
R⁶ - für Alkyl, Alkenyl oder Alkinyl steht
und
X - für Halogen steht,
alkyliert,
im Fall der Herstellung von Säuren die Ester verseift und gegebenenfalls durch Zugabe von Basen die Salze herstellt.

2. Verfahren nach Anspruch 1 zur Herstellung von gegebenenfalls α-substituierten [4-(Chinolin-2-yl-methoxy)phenyl]-essigsäuren und -estern
worin
R¹ - für Wasserstoff, C₁-C₆-Alkyl, Benzyl, Phenyl, oder
- für eine Gruppe der Formel
-CH₂-CO₂-R³
steht,
wobei
R³ - für Wasserstoff, C₁-C₆-Alkyl, Phenyl oder Benzyl steht
und
R² - für Wasserstoff, C₁-C₆-Alkyl, verzweigtes C₁-C-₆Alkyl, C₂-C₆-Alkenyl, verzweigtes C₂-C₆-Alkenyl, C₂-C₆-Alkinyl oder verzweigtes C₂-C₆-Alkinyl steht,
sowie deren Salze.

3. Verfahren nach Anspruch 1 zur Herstellung von gegebenenfalls α-substituierten [4-(Chinolin-2-yl-methoxy)phenyl]-essigsäuren und -estern nach den Ansprüchen 1 und 2,
worin
R¹ - für Wasserstoff, Methyl, Ethyl, Benzyl, Phenyl, oder
- für eine Gruppe der Formel
-CH₂-CO₂-R³
steht,
wobei
R³ - für Wasserstoff, Methyl, Ethyl, Phenyl oder Benzyl steht
und
R² - für Wasserstoff, Methyl, Ethyl, Propyl, Butyl, Pentyl, Hexyl, Isopropyl, Isobutyl, Isopentyl, 2-Butenyl, 2-Pentenyl, Allyl, 3,3-Dimethylallyl, 2-Butinyl, 2-Propinyl, 3-Pentinyl, 1-Methyl-2-butinyl oder 3-Hexinyl steht,
sowie deren Salze.

4. Verfahren nach Anspruch 1 zur Herstellung von 4-(Chinolin-2-yl-methoxy)phenyl-essigsäure der Formel 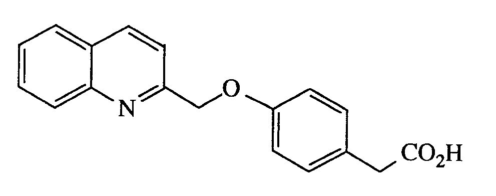 und deren Salze.

5. Verfahren nach Anspruch 1, dadurch gekennzeichnet, daß man in einem Temperaturbereich von 0°C bis 150°C arbeitet.

## Claims (Claims for the following Contracting State(s): AT, BE, CH, DE, FR, GB, GR, IT, LI, NL, SE)

1. Optionally α-substituted [4-(quinolin-2-yl-methoxy)-phenyl]-acetic acids and esters of the general formula 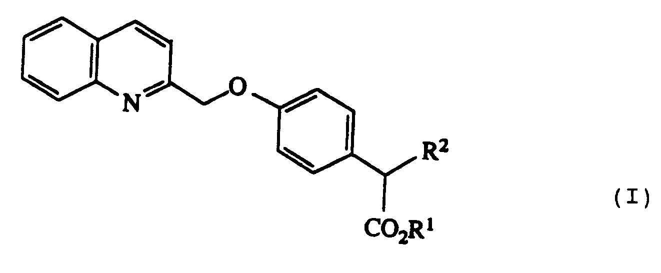 in which
R¹ - represents hydrogen, alkyl having 1 to 6 carbon atoms, an aralkyl radical having 1 to 6 carbon atoms in the aliphatic moiety and 6 to 12 carbon atoms in the aromatic moiety or aryl having 6 to 12 carbon atoms,
or
- represents a group of the formula
-CH₂-CO₂-R³,
where
R³ - represents hydrogen, alkyl having 1 to 6 carbon atoms, an aralkyl radical having 1 to 6 carbon atoms in the aliphatic moiety and 6 to 12 carbon atoms in the aromatic moiety or aryl having 6 to 12 carbon atoms,
and
R² - represents hydrogen, alkyl, having 1 to 6 carbon atoms, alkenyl having 2 to 6 carbon atoms or alkinyl having 2 to 6 carbon atoms,
and their salts.

2. Optionally α-substituted [4-(quinolin-2-yl-methoxy)-phenyl]-acetic acids and esters according to Claim 1,
wherein
R¹ - represents hydrogen, C₁-C₆-alkyl, benzyl or phenyl, or
- represents a group of the formula
-CH₂-CO₂-R³,
where
R³ - represents hydrogen, C₁-C₆-alkyl, phenyl or benzyl
and
R² represents hydrogen, C₁-C₆-alkyl, branched C₁-C₆-alkyl, C₂-C₆-alkenyl, branched C₂-C₆-alkenyl, C₂-C₆-alkinyl or branched C₂-C₆-alkinyl,
and their salts

3. Optionally α-substituted [4-(quinolin-2-yl-methoxy)-phenyl]-acetic acids and eaters according to Claims 1 and 2,
wherein
R¹ - represents hydrogen, methyl, ethyl, benzyl or phenyl, or
- represents a group of the formula
-CH₂-CO₂-R³,
where
R³ - represents hydrogen, methyl, ethyl, phenyl or benzyl,
and
R² - represents hydrogen, methyl, ethyl, propyl, butyl, pentyl, hexyl, isopropyl, isobutyl, isopentyl, 2-butenyl, 2-pentenyl, allyl, 3,3-dimethylallyl, 2-butinyl, 2-propinyl, 3-pentinyl, 1-methyl-2-butinyl or 3-hexinyl,
and their salts

4. 4-(Quinolin-2-yl-methoxy)phenyl-acetic acid of the formula 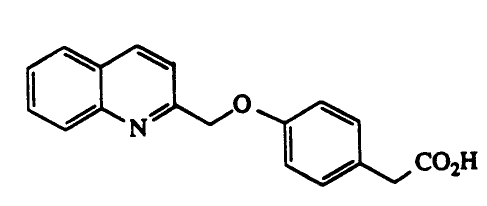 and its salts according to Claims 1 to 3.

5. Optionally α-substituted [4-(quinolin-2-yl-methoxy)-phenyl]-acetic acids and esters according to Claims 1 to 3 for therapeutic treatment.

6. Process for the preparation of optionally α-substituted [4-(quinolin-2-yl-methoxy)phenyl]-acetic acids and esters of the formula 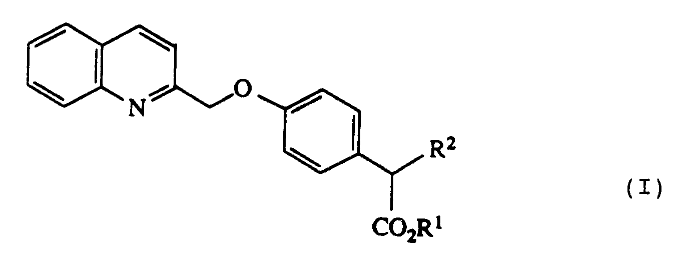 in which R¹ and R² have the meaning given in Claim 1, characterised in that
[A] phenols of the general formula (II) 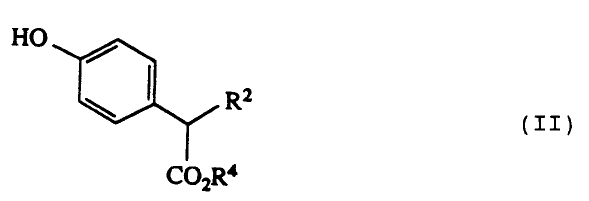 in which
R² has the abovementioned meaning
and
R⁴ - represents alkyl, arylalkyl or aryl, or
- represents a group of the formula
-CH₂-CO₂-R⁵
where
R⁵ - represents alkyl, aralkyl or aryl,
are reacted with 2-halogenomethylquinoline of the formula (III) 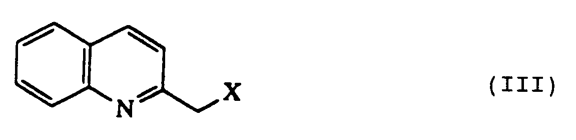 in which
X represents halogen,
and in the case of the acids the esters are hydrolysed,
or in that
[B] esters of the general formula (IV) 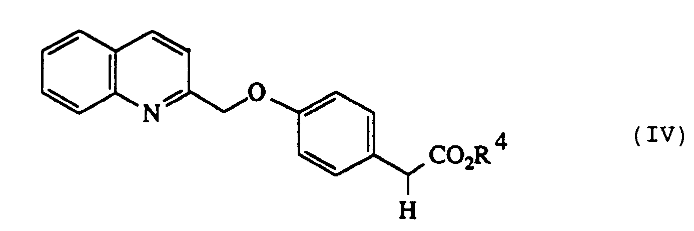 in which
R⁴ has the abovementioned meaning,
are alkylated with halides of the general formula (V)
R⁶-X V
in which
R⁶ represents alkyl, alkenyl or alkinyl
and
X represents halogen,
in the case of the preparation of acids, the esters are hydrolysed and, if desired, the salts are prepared by addition of bases.

7. Medicaments, containing optionally α-substituted [4-quinolin-2-yl-methoxy)phenyl]-acetic acids and esters according to Claims 1 to 3.

8. Medicaments according to Claim 7, containing 0.5 to 90% by weight of optionally α-substituted [4-quinolin-2-yl-methoxy)phenyl]-acetic acids and esters relative to the preparation.

9. Use of optionally α-substituted [4-(quinolin-2-yl-methoxy)phenyl]-acetic acids and esters according to Claims 1 to 3 for the production of medicaments.

10. Use according to Claim 9 for the preparation of lipoxygenase inhibitors.

## Claims (Claims for the following Contracting State(s): ES)

1. Process for the preparation of optionally α-substituted [4-(quinolin-2-yl-methoxy)phenyl]-acetic acids and esters of the general formula 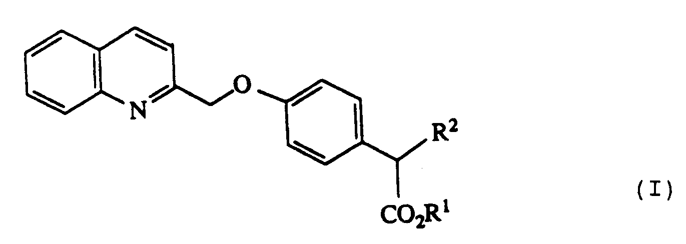 in which
R¹ - represents hydrogen, alkyl having 1 to 6 carbon atoms, an aralkyl radical having 1 to 6 carbon atoms in the aliphatic moiety and 6 to 12 carbon atoms in the aromatic moiety or aryl having 6 to 12 carbon atoms,
or
- represents a group of the formula
-CH₂-CO₂-R³,
where
R³ - represents hydrogen, alkyl having 1 to 6 carbon atoms, an aralkyl radical having 1 to 6 carbon atoms in the aliphatic moiety and 6 to 12 carbon atoms in the aromatic moiety or aryl having 6 to 12 carbon atoms,
and
R² - represents hydrogen, alkyl, having 1 to 6 carbon atoms, alkenyl having 2 to 6 carbon atoms or alkinyl having 2 to 6 carbon atoms,
and their salts, characterised in that
[A] phenols of the general formula (II) 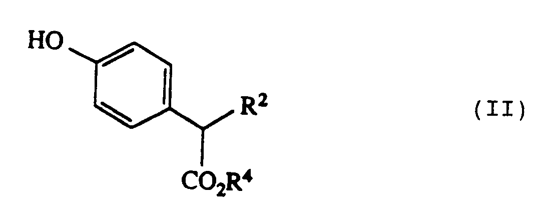 in which
R² has the abovementioned meaning
and
R⁴ -represents alkyl, arylalkyl or aryl, or
-represents a group of the formula
-CH₂-CO₂-R⁵
where
R⁵ - represents alkyl, aralkyl or aryl,
are reacted with 2-halogenomethylquinoline of the formula (III) 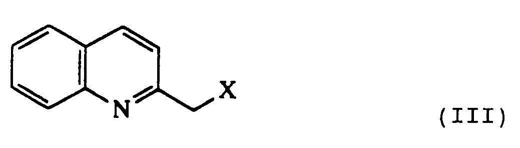 in which
X represents halogen,
and in the case of the acids the esters are hydrolysed,
or in that
[B] esters of the general formula (IV) 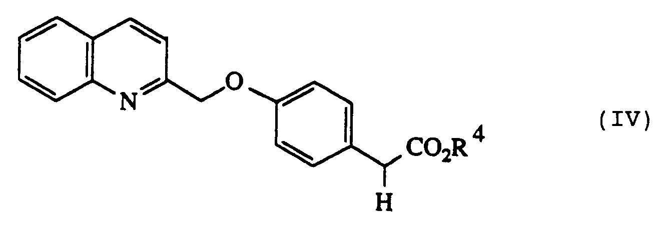 in which
R⁴ has the abovementioned meaning,
are alkylated with halides of the general formula (V)
R⁶-X V
in which
R⁶ -represents alkyl, alkenyl or alkinyl
and
X - represents halogen,
in the case of the preparation of acids, the esters are hydrolysed and, if desired, the salts are prepared by addition of bases.

2. Process according to Claim 1 for the preparation of optionally α-substituted [4-(quinolin-2-yl-methoxy)-phenyl]-acetic acids and esters
wherein
R¹ - represents hydrogen, C₁-C₆-alkyl, benzyl or phenyl, or
- represents a group of the formula
-CH₂-CO₂-R³,
where
R³ - represents hydrogen, C₁-C₆-alkyl, phenyl or benzyl
and
R² represents hydrogen, C₁-C₆-alkyl, branched C₁-C₆-alkyl, C₂-C₆-alkenyl, branched C₂-C₆-alkenyl, C₂-C₆-alkinyl or branched C₂-C₆-alkinyl,
and their salts

3. Process according to Claim 1 for the preparation of optionally α-substituted [4-(quinolin-2-yl-methoxy)-phenyl]-acetic acids and esters
wherein
R¹ - represents hydrogen, methyl, ethyl, benzyl or phenyl, or
- represents a group of the formula
-CH₂-CO₂-R³,
where
R³ - represents hydrogen, methyl, ethyl, phenyl or benzyl,
and
R² - represents hydrogen, methyl, ethyl, propyl, butyl, pentyl, hexyl, isopropyl, isobutyl, isopentyl, 2-butenyl, 2-pentenyl, allyl, 3,3-dimethylallyl, 2-butinyl, 2-propinyl, 3-pentinyl, 1-methyl-2-butinyl or 3-hexinyl,
and their salts

4. Process according to Claim 1 for the preparation of [4-(quinolin-2-yl-methoxy)phenyl]-acetic acid of the formula 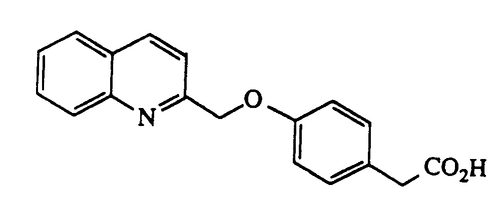 and their salts.

5. Process according to Claim 1, characterised in that it is carried out in a temperature range from 0°C to 150°C.

## Revendications (Revendications pour l'(les) Etat(s) contractant(s) suivant(s): AT, BE, CH, DE, FR, GB, GR, IT, LI, NL, SE)

1. Acides et esters [4-(quinoléine-2-yl-méthoxy)-phényl]-acétiques éventuellement substitués en alpha, de formule générale 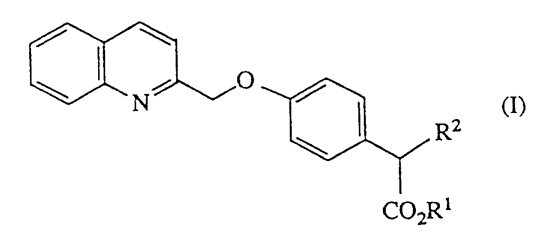 dans laquelle
R¹ représente l'hydrogène, un groupe alkyle en C₁-C₆, un groupe aralkyle contenant 1 à 6 atomes de carbone dans la partie aliphatique et 6 à 12 atomes de carbone dans la partie aromatique, un groupe aryle en C₆-C₁₂,
ou bien
- un groupe de formule
-CH₂-CO₂-R³
dans laquelle
R³ représente l'hydrogène, un groupe alkyle en C₁-C₆, un groupe aralkyle contenant 1 à 6 atomes de carbone dans la partie aliphatique et 6 à 12 atomes de carbone dans la partie aromatique, ou un groupe aryle en C₆-C₁₂, et
R² représente l'hydrogène, un groupe alkyle en C₁-C₆, alcényle en C₂-C₆ ou alcynyle en C₂-C₆,
et leurs sels

2. Acides et esters [4-(quinoléine-2-yl-méthoxy)-phényl]-acétiques éventuellement substitués en alpha selon la revendication 1,
pour lesquels
R¹ représente l'hydrogène, un groupe alkyle en C₁-C₆, benzyle, phényle, ou bien
- un groupe de formule
-CH₂-CO₂-R³
dans laquelle
R³ représente l'hydrogène, un groupe alkyle en C₁-C₆, phényle ou benzyle, et
R² représente l'hydrogène, un groupe alkyle en C₁-C₆, un groupe alkyle ramifié en C₁-C₆, un groupe alcényle en C₂-C₆, un groupe alcényle ramifié en C₂-C₆, un groupe alcynyle en C₂-C₆, un groupe alcynyle ramifié en C₂-C₆,
et leurs sels.

3. Acides et esters [4-(quinoléine-2-yl-méthoxy)-phényl]-acétiques éventuellement substitués en alpha selon les revendications 1 et 2
pour lesquels
R¹ représente l'hydrogène, un groupe méthyle, éthyle, benzyle, phényle ou bien,
- un groupe de formule
-CH₂-CO₂-R³
dans laquelle
R³ représente l'hydrogène, un groupe méthyle, éthyle, phényle ou benzyle, et
R² représente l'hydrogène, un groupe méthyle, éthyle, propyle, butyle, pentyle, hexyle, isopropyle, isobutyle, isopentyle, 2-butényle, 2-pentényle, allyle, 3,3-diméthylallyle, 2-butynyle, 2-propynyle, 3-pentynyle, 1-méthyl-2-butynyle ou 3-hexynyle,
et leurs sels.

4. Acide 4-(quinoléine-2-yl-méthoxy)-phénylacétique de formule 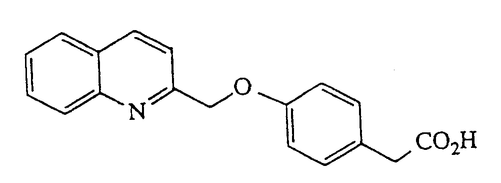 et ses sels selon les revendications 1 à 3.

5. Acides et esters [4-(quinoléine-2-yl-méthoxy)-phényl]-acétiques éventuellement substitués en alpha selon les revendications 1 à 3, pour l'utilisation en thérapeutique.

6. Procédé de préparation des acides et esters [4-(quinoléine-2-yl-méthoxy)-phényl]-acétiques éventuellement substitués en alpha, de formule 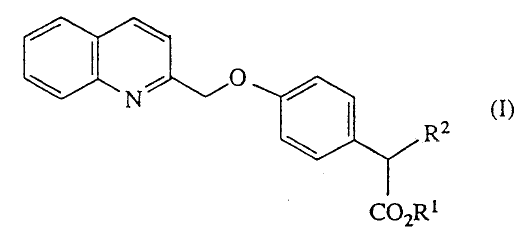 dans laquelle R¹ et R² ont les significations indiquées dans la revendication 1, caractérisé en ce que :
(A) on fait réagir des phénols de formule générale II 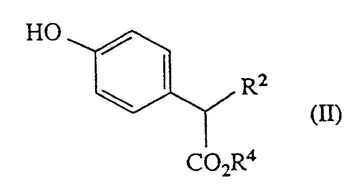 dans laquelle R² a les significations indiquées ci-dessus, et
R⁴ représente un groupe alkyle, arylalkyle, aryle, ou bien
- un groupe de formule
-CH₂-CO₂-R⁵
dans laquelle R⁵ représente un groupe alkyle, aralkyle ou aryle,
avec une 2-halogénométhylquinoléine de formule III 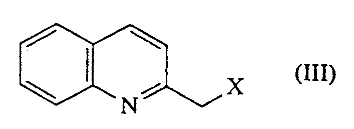 dans laquelle X représente un halogène,
et, dans le cas des acides, on saponifie les esters,
ou bien
(B) on alkyle des esters de formule générale IV 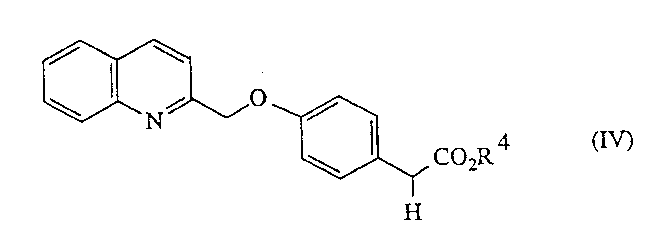 dans laquelle R⁴ a les significations indiquées ci-dessus,
à l'aide d'halogénures de formule générale V
R⁶-X (V)
dans laquelle
R⁶ représente un groupe alkyle, alcényle ou alcynyle, et
X représente un halogène,
et, dans le cas où on prépare les acides, on saponifie les esters, et le cas échéant on prépare les sels par addition de bases.

7. Médicament contenant des acides et des esters [4-(quinoléine-2-yl-méthoxy)-phényl]-acétiques éventuellement substitués en alpha selon les revendications 1 à 3.

8. Médicament selon la revendication 7, contenant 0,5 à 90 % de son poids d'acides et esters [4-(quinoléine-2-yl-méthoxy)-phényl]-acétiques éventuellement substitués en alpha.

9. Utilisation des acides et esters [4-(quinoléine-2-yl-méthoxy)-phényl]-acétiques éventuellement substitués en alpha selon les revendications 1 à 3 pour la préparation de médicaments.

10. Utilisation selon la revendication 9, pour la préparation d'inhibiteurs de la lipoxygénase.

## Revendications (Revendications pour l'(les) Etat(s) contractant(s) suivant(s): ES)

1. Procédé de préparation d'acides et esters [4-(quinoléine-2-yl-méthoxy)-phényl]-acétiques éventuellement substitués en alpha, de formule générale 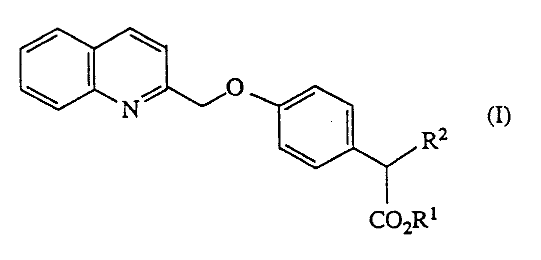 dans laquelle R¹ représente l'hydrogène, un groupe alkyle en C₁-C₆, un groupe aralkyle contenant 1 à 6 atomes de carbone dans la partie aliphatique et 6 à 12 atomes de carbone dans la partie aromatique, un groupe aryle en C₆-C₁₂,
ou bien
- un groupe de formule
-CH₂-CO₂-R³
dans laquelle
R³ représente l'hydrogène, un groupe alkyle en C₁-C₆, un groupe aralkyle contenant 1 à 6 atomes de carbone dans la partie aliphatique et 6 à 12 atomes de carbone dans la partie aromatique, ou un groupe aryle en C₆-C₁₂, et
R² représente l'hydrogène, un groupe alkyle en C₁-C₆, alcényle en C₂-C₆ ou alcynyle en C₂-C₆,
et de leurs sels, caractérisé en ce que :
(A) on fait réagir des phénols de formule générale II 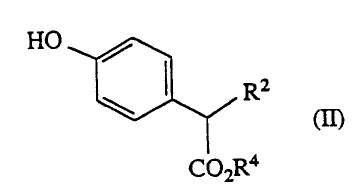 dans laquelle R² a les significations indiquées ci-dessus, et
R⁴ représente un groupe alkyle, arylalkyle, aryle, ou bien
- un groupe de formule
-CH₂-CO₂-R⁵
dans laquelle R⁵ représente un groupe alkyle, aralkyle ou aryle,
avec une 2-halogénométhylquinoléine de formule III 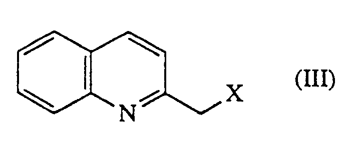 dans laquelle X représente un halogène,
et, dans le cas des acides, on saponifie les esters,
ou bien
(B) on alkyle des esters de formule générale IV 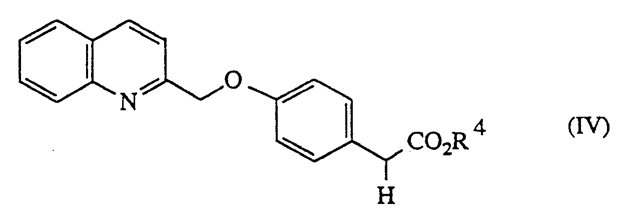 dans laquelle R⁴ a les significations indiquées ci-dessus,
à l'aide d'halogénures de formule générale V
R⁶-X (V)
dans laquelle
R⁶ représente un groupe alkyle, alcényle ou alcynyle, et
X représente un halogène,
et, dans le cas où on prépare les acides, on saponifie les esters, et le cas échéant on prépare les sels par addition de bases.

2. Procédé selon la revendication 1, pour la préparation d'acides et esters [4-(quinoléine-2-yl-méthoxy)-phényl]-acétiques éventuellement substitués en alpha pour lesquels
R¹ représente l'hydrogène, un groupe alkyle en C₁-C₆, benzyle, phényle, ou bien
- un groupe de formule
-CH₂-CO₂-R³
dans laquelle
R³ représente l'hydrogène, un groupe alkyle en C₁-C₆, phényle ou benzyle, et
R² représente l'hydrogène, un groupe alkyle en C₁-C₆, un groupe alkyle ramifié en C₁-C₆, un groupe alcényle en C₂-C₆, un groupe alcényle ramifié en C₂-C₆, un groupe alcynyle en C₂-C₆, un groupe alcynyle ramifié en C₂-C₆,
et leurs sels.

3. Procédé selon la revendication 1, pour préparer des acides et esters [4-(quinoléine-2-yl-méthoxy)-phényl]-acétiques éventuellement substitués en alpha selon les revendications 1 et 2, pour lesquels
R¹ représente l'hydrogène, un groupe méthyle, éthyle, benzyle, phényle ou bien,
- un groupe de formule
-CH₂-CO₂-R³
dans laquelle
R³ représente l'hydrogène, un groupe méthyle, éthyle, phényle ou benzyle, et
R² représente l'hydrogène, un groupe méthyle, éthyle, propyle, butyle, pentyle, hexyle, isopropyle, isobutyle, isopentyle, 2-butényle, 2-pentényle, allyle, 3,3-diméthylallyle, 2-butynyle, 2-propynyle, 3-pentynyle, 1-méthyl-2-butynyle ou 3-hexynyle,
et leurs sels.

4. Procédé selon la revendication 1, pour la préparation de l'acide 4-(quinoléine-2-yl-méthoxy)-phénylacétique de formule 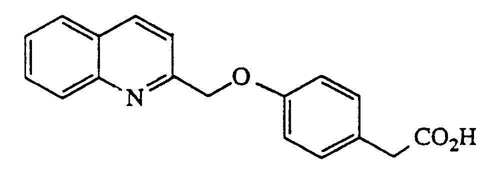 et de ses sels.

5. Procédé selon la revendication 1, caractérisé en ce que l'on opère dans l'intervalle de température de 0 à 150°C.
